# EUROPÄISCHE PATENTSCHRIFT

(11) **EP 3 041 404 B1**
(45) Veröffentlichungstag und Bekanntmachung des Hinweises auf die Patenterteilung: **23.08.2017**
(21) Anmeldenummer: 14841420.4
(22) Anmeldetag: 03.09.2014
(51) Int. Cl.: A61B 5/00, A61B 5/053

(54) **VORRICHTUNG ZUR MESSUNG VON PHYSIOLOGISCHEN WERTEN**
DEVICE FOR MEASURING PHYSIOLOGICAL VALUES
DISPOSITIF DE MESURE DE VALEURS PHYSIOLOGIQUES

(30) Priorität: 05.09.2013 DE 102013014982
(43) Veröffentlichungstag der Anmeldung: 13.07.2016
(73) Patentinhaber: seca ag, 4153 Reinach BL 1 (CH)
(72) Erfinder: RHEIN, Detlef, 22083 Hamburg (DE); GARTHOFF, Till, 20251 Hamburg (DE); BROWN, Julian, Bristal BS29HE (GB)
(74) Vertreter: Patentanwälte Klickow & Partner Partnerschaftsgesellschaft mbB
(86) Internationale Anmeldenummer: PCT/IB2014/002093
(87) Internationale Veröffentlichungsnummer: WO 2015/044767

(56) Entgegenhaltungen:
- EP-A1- 1 627 597
- US-A1- 2010 137 704
- US-A1- 2010 324 433

## Beschreibung

Die Erfindung betrifft eine Vorrichtung zur messtechnischen Kontaktherstellung zwischen einem Patienten und einem Messgerät, aufweisend wenigstens ein Messmittel zur Anbindung an den Patienten und wenigstens eine Leitung, an der das Messmittel festgelegt ist, wobei die Leitung derart ausgebildet ist, dass die durch das Messmittel erfassten Messgrößen an ein Messgerät leitbar sind sowie bei der eine Matte mit der wenigstens einen Leitung derart verbunden ist, dass wenigstens teilweise ein interner Leitungsabschnitt gebildet ist,

Die Erfindung betrifft darüber hinaus ein Messsystem zur Messung von physiologischen Werten.

Schließlich betrifft die Erfindung die Verwendung der Vorrichtung zur messtechnischen Kontaktherstellung zwischen einem Subjekt und einem Messgerät.

Es ist bekannt, dass zur Messung von Körpereigenschaften und Körperfunktionen von Subjekten, z.B. menschlichen Personen oder Tieren, mobile oder stationäre Messgeräte eingesetzt werden. Bei den zu messenden Eigenschaften oder Funktionen handelt es sich häufig um Bio- bzw. Körperimpedanzen, Herzfunktionen, Nervenfunktionen oder Elektrokardiogrammen. Für diese Messungen ist es erforderlich, dass das Messgerät mit dem zu messenden Subjekt verbunden wird. Im Wesentlichen sind für die Verbindung zwei verschiedene Varianten bekannt.

Zum einen kann das Messgerät Kontaktstellen, beispielsweise Griff- oder Standflächen aufweisen, mit deren Hilfe durch Berührung des Subjektes die erforderliche Verbindung hergestellt wird. Eine derartige Ausgestaltung des Kontaktes zwischen Subjekt und Messgerät zeigt beispielsweise die DE 10 2010 023 122 A1. Es handelt sich hierbei um eine Vorrichtung, die den Kontakt zwischen einem stehenden Subjekt und dem Messgerät herstellt.

Ausgangspunkt der Erfindung ist eine zweite bekannte Vorrichtung, welche eine Kontaktherstellung zwischen dem Messgerät und Subjekt durch eine oder mehrere Leitungen, die Schläuche oder Kabel sein können, realisiert. In der Regel sind die Leitungen jeweils endseitig mit dem Messgerät und über Messmittel, wie z.B. Elektroden, mit dem Subjekt verbunden. Die meisten Körperfunktionsmessungen erfordern mehrere Kontaktstellen am Subjekt, häufig an den Extremitäten wie Armen und Beinen und dort wiederum vielfach an Händen und oder Füßen. Dabei ist häufig jeder endseitigen Leitung genau eine Extremität zugewiesen. Das bedeutet, dass die mehreren Messstellen des Subjektes mit den jeweils zugeordneten Messmitteln und Leitungen korrekt angeschlossen werden müssen. Auch sind häufig für eine Messstelle am Subjekt zwei oder mehrere Leitungen erforderlich. Eine weitere Variante ist, dass sich eine Leitung in mehrere Teilleitungen aufteilt und endseitige Messmittel aufweist, die an dem Subjekt angebracht werden. Die Kontaktherstellung wird bei dieser Vorrichtung bevorzugt bei liegenden oder sitzenden Subjekten realisiert.

Ein Schwachpunkt der bekannten Vorrichtung ist, dass mit der Kontaktherstellung des Patienten als eines der möglichen Subjekte ein äußerst unangenehmes Gefühl des "Verkabeltseins" entsteht. Mögliche Folgen können sein, dass der Patient die Messuntersuchung abbricht oder gar nicht erst in Erwägung zieht.

Weiterhin ist nachteilig, dass die korrekte Zuordnung der an den Leitungen endseitig angebrachten Elektroden zu der jeweiligen Extremität nur durch optisch oder haptisch zu erfassende Positionierungsanweisungen an das Bedienpersonal realisiert werden kann. Es besteht die Gefahr, dass die Positionierung der Elektroden an der falschen Extremität, d.h. linke oder rechte Hand, linker oder rechter Fuß bzw. zwischen Händen und Füßen vorgenommen wird - insbesondere wenn das Bedienpersonal die Anweisung nicht oder falsch wahrnimmt

Nachteilig an der bekannten Vorrichtung ist darüber hinaus, dass die Verstauung der Leitungen für den Transport oder die Lagerung nach Messungsende umständlich und langwierig ist. Auch besteht die latente Gefahr der Beschädigung. Eine im Stand der Technik bekannte Lösung des Beschädigungsproblems bei Verstauung besteht darin, dass die Leitungen durch eine Ummantelung vor Abknicken oder Verdrillen geschützt werden sollen. Die Ummantelung schützt jedoch nicht zuverlässig vor diesen Beschädigungen und verursacht eine Versteifung der Leitungen, was die Handhabung verschlechtert und die Bewegungsfreiheit des Subjektes nach Anlegen der mit den Leitungen verbundenen Messmittel einschränkt. Auch verhindert die Ummantelung der Leitungen durch die dadurch verursachte Vergrößerung des minimal möglichen Aufrollradius der Leitungen, dass diese mit kleinem Packmaß verstaut werden können. Ein weiterer Nachteil dieser bekannten Vorrichtung ist, dass die Leitungen zum verknoten, verdrehen oder verheddern neigen. Dadurch wird die Handhabung zusätzlich erschwert.

Aus der US 2010/137704 A1 ist bereits eine Vorrichtung zur messtechnischen Kontaktherstellung zwischen einem Patienten und einem Messgerät bekannt. Die Vorrichtung besitzt Messmittel und Leitungen. Ein Teil der Leitungen erstreckt sich in einer Messmatte.

Eine ähnliche Vorrichtung wird auch in der US 2010/324433 A1 beschrieben. Eine weitere ähnliche Vorrichtung ist auch ist auch aus der EP 1627597 A1 bekannt.

Es ist Aufgabe der Erfindung, eine Vorrichtung der eingangs beschriebenen Art zu schaffen, die eine generelle Verbesserung der Handhabung und Transportsicherheit bietet und die die eingangs beschriebenen Nachteile vermindert.

Diese Aufgabe wird gelöst dadurch, dass die Messmatte als Elektroden zur Durchführung einer Bioimpedanzmessung ausgebildet ist und dass mindestens eine Elektrode für jede Hand des Patienten und mindestens eine Elektrode für jeden Fuß des Patienten angeordnet ist.

Der mit der Erfindung erzielte Vorteil besteht darin, dass durch die wenigstens teilweise Verbindung der Leitungen mit einer Matte das äußerst unangenehme Gefühl des "Verkabeltseins" reduziert oder verhindert wird. Die Zuordnungssicherheit der jeweiligen Messmittel zu den Extremitäten wird unterstützt dadurch, dass durch die Lage der Matte auf oder unter dem Subjekt das jeweils örtlich nächstgelegene Messmittel, z.B. eine Elektrode, verwendet wird. Die sichere Verstauung mit kleinem Packmaß wird durch die Eigenschaft der Matte, zusammenlegbar zu sein gefördert.
Weitere vorteilhafte Ausgestaltungen der Erfindung sind in den abhängigen Ansprüchen beschrieben.

Infolge der Verwendung einer vorzugsweise elastischen, jedenfalls aber zumindest zusammenlegbaren Matte, welche wenigstens teilweise die gemäß dem Stand der Technik bekannten Leitungen als Mittel zur Kontaktherstellung zwischen zu messendem Subjekt und Messgerät ergänzt, werden die vorteilhaften Wirkungen erzielt. Die Elektroden können auch als Handelektroden vorhanden sein.

Erfindungsgemäß vermeidet die Anordnung der Elektroden auf der Matte das Aufkleben einzelner Elektroden auf die Haut des Patienten. Statt der Vielzahl von einzelnen zu jeder der Elektroden führenden Kabel wird nur ein gegebenenfalls aus mehreren Leitungen bestehendes Kabel zur Verbindung der Matte mit dem Messgerät verwendet.

Zur Vermeidung von Leitungen zur Energieversorgung von Bauelementen auf der Matte kann die Matte mit einer Batterie oder einem wiederaufladbaren Akkumulator ausgestattet werden. Ein Aufladen des Akkumulators kann beispielsweise bei einer Aufbewahrung der Matte im Bereich einer dafür vorgesehenen Halterung erfolgen. Zusätzlich kann die Matte mit Bedienknöpfen und Einrichtungen zur Darstellung eines aktuellen Zustandes ausgestattet sein, z. B. Start, Stopp, niedriger Ladezustand.

Bei einer geeigneten zusammenlegbaren Ausbildung der Matte können die Kabel gegen ein Herausfallen bei einem Transport und bei der Aufbewahrung gesichert sein.

Die erfindungsgemäße Vorrichtung zur Messung von physiologischen Werten besteht in einer ersten Ausführungsform aus einer Matte, an welcher die Leitungen wenigstens teilweise festgelegt sind oder diese aufnimmt. An den Leitungen sind endseitig Messmittel festgelegt. Die Leitungen weisen einen internen Abschnitt auf, d.h. ein Leitungsabschnitt verläuft innerhalb der Matte oder ist an der Außenfläche der Matte festgelegt, und einen externen Abschnitt auf, d.h. es ist ein Leitungsabschnitt außerhalb der Matte.

Im Fall des Leitungsverlaufes als internen Abschnitt innerhalb der Matte ist die Matte in ihrem innern als Hohlraum ausgebildet derart, dass der interne Leitungsabschnitt aufgenommen werden kann. Dies kann beispielsweise dadurch realisiert werden, dass die Matte aus zwei biegsamen plattenartigen bzw. flächigen Halbzeugen besteht, die an ihren jeweiligen Außenrändern miteinander verbunden sind.

Bei der Verwendung eines aufschäumbaren oder gießfähigen Werkstoffes als Ausgangsmaterial für die Matte kann der interne Abschnitt der Leitung auch direkt in den Mattenwerkstoff eingebettet werden. In einer bevorzugten Ausführungsform können die Leitungen derart angeordnet sein, dass der Übergang vom internen zum externen Leitungsabschnitt in den Eckenbereichen einer viereckig oder rechteckig ausgebildeten Matte erfolgt. Eine weitere Leitung mit internem und oder externem Abschnitt kann vorhanden sein um die Vorrichtung mit dem Messgerät zu verbinden.

In einer zweiten bevorzugten Ausführungsform substituiert die Matte die gemäß dem Stand der Technik verwendeten Leitungen vollständig, d.h. es liegen nur Leitungen mit internem Abschnitt vor. Wird die Erfindung in dieser Form ausgestaltet, sind die Messmittel direkter Bestandteil der Matte oder an der Matte festgelegt. Als Messmittel können zum Beispiel herkömmliche Klebeelektroden, Auflageelektroden, Elektrodengriffe, federgespannte Klemmelektroden oder Fußkontaktelektroden verwendet werden. Bei der Verwendung von Elektrodengriffen und Fußkontaktelektroden ist das zu messenden Subjekt aufgefordert, die entsprechenden Kontakte mit den Extremitäten durch Berührung herzustellen. Im Fall der Elektrodengriffe sind diese mit den Händen zu umfassen. Durch Auflegen der Füße auf die Fußkontaktelektroden wird die Verbindung mit diesen hergestellt. Im Fall der Verwendung von Klebeelektroden ist durch Aufkleben an Händen bzw. Füßen die Verbindung herzustellen.

Es ist weiterhin möglich, dass das Messgerät extern, d.h. außerhalb und beabstandet zur Matte angeordnet oder auf der Matte festgelegt oder integraler Bestandteil innerhalb der Matte ist. Die internen Leitungsabschnitte können innerhalb der Matte in einem Punkt zusammenlaufen, der sich vorzugsweise mittig der Mattenfläche befindet.

Die Matte kann auf ein sitzendes oder liegendes Subjekt aufgelegt werden. Auch kann die Matte als Unterlage für ein sitzendes oder liegendes Subjekt Verwendung finden. Wird die erfindungsgemäße Matte als Unterlage unter das Subjekt ausgestaltet oder verwendet reduziert sich das äußerst unangenehme Gefühl des "Verkabeltseins" zusätzlich, das Subjekt nimmt die Leitungsverbindung praktisch kaum noch war.

Die erfindungsgemäße Vorrichtung unterstützt die sichere Zuordnung der Messmittel an die jeweilige Extremität. Auch bei beliebiger Ausgestaltung der Mattenform kann das Bedienpersonal die Matte relativ zum Subjekt derart positionieren, dass immer eine Messmitteleinrichtung der jeweiligen Extremität am nächsten ist. Somit ist einzig durch die Lage der Matte relativ zum zu messenden Subjekt eine Messmittelzuordnung realisiert. Die korrekte Lage der Matte relativ zum zu messenden Subjekt kann dem Bedienpersonal dadurch angezeigt werden, dass auf der Matte ein Richtungshinweis oder Positionieranweisung angebracht ist. Auch könnte die Körperkontur auf der Matte abgebildet sein, welche mit der Matte in Kontakt zu bringen ist. Ist die erfindungsgemäße Matte in der zweiten bevorzugten Ausführungsform und unter Verwendung von Elektrodengriffen und Fußkontaktelektroden realisiert, ergibt sich zum einen die korrekte Zuordnung der Messmittel zu Händen und Füßen durch die jeweiligen Elektrodengriffe bzw. Fußkontaktelektroden und die Links-rechts-Zuordnung durch die Ausrichtung der Matte bezüglich Vorderseite oder Rückseite.

Um die sichere Verstauung mit kleinem Packmaß zu unterstützen ist die Matte derart ausgebildet, dass sie zusammenlegbar ist. Bei der Verwendung von Leitungen mit internem und externem Abschnitt ist die Matte derart zusammenlegbar, dass die externen Leitungsabschnitte innerhalb der zusammen gelegten Matte aufgenommen werden können und gegen herausfallen gesichert sind.

Die Erfindung wird nachfolgend mit Bezug auf die Figuren erläutert. Im Einzelnen zeigen
- Fig. 1: eine Vorrichtung zur Messung von physiologischen Werten gemäß dem Stand der Technik,
- Fig. 2: die schematische Darstellung der Vorrichtung zur Messung von physiologischen Werten in einer ersten Ausführungsform,
- Fig. 3: die Verwendung der Vorrichtung zur Messung von physiologischen Werten in einer ersten Ausführungsform,
- Fig. 4: die schematische Darstellung der Vorrichtung zur Messung von physiologischen Werten besteht in einer ersten Ausführungsform in zusammengerolltem Zustand und
- Fig. 5: die zweite Ausführungsform, in der die Matte 5 die gemäß dem Stand der Technik verwendeten Leitungen 3 vollständig substituiert - Messmittel direkt an der Matte festgelegt.

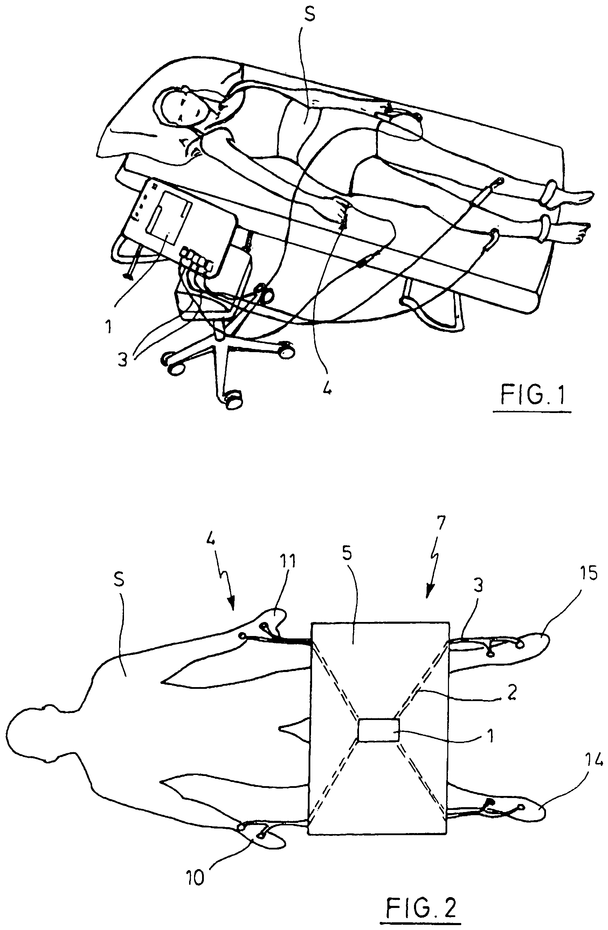

Fig. 1 zeigt eine Vorrichtung zur Messung von physiologischen Werten gemäß dem Stand der Technik. Das mobile Messgerät 1 ist mit den zu messenden Subjekt S durch mehrere Leitungen 3 und Messmittel 4 verbunden, d.h. die Kontaktherstellung zwischen Messgerät 1 und zu messenden Subjekt erfolgt durch Leitungen. Die Messmittel 4, die endseitig an den Leitungen angebracht sind, sind sowohl an den Händen als auch an den Füßen des Objektes festgelegt. Es ist ersichtlich, dass diese Art der Kontaktherstellung zwischen Subjekt S und Messgerät 1 das Gefühl des Verkabeltseins verstärkt, die Messmittelzuordnung zu der jeweiligen Extremität fehlerträchtig ist und die Leitungen verknoten oder verheddern können. Weiterhin ist im Stand der Technik bekannt, dass mehr oder auch weniger Leitungen 3, als die Anzahl wie in Fig. 1 dargestellt Verwendung finden.

Fig. 2 zeigt schematisch eine erste Ausführungsform der erfindungsgemäßen Vorrichtung 7 zur Kontaktherstellung zwischen Messmittel 3 und Messgerät 7. Sie besteht aus einer Matte 5, welche die Leitungen 2 zwischen den Messmittel 3 und dem Messgerät 1 wenigstens teilweise aufnimmt oder ergänzt. Die Matte 5 weist Leitungen 3 auf, an denen endseitig Messmittel 4 festgelegt sind. Die Leitungen 2, 3 weisen einen internen Abschnitt 2 auf, d.h. ein Leitungsabschnitt verläuft innerhalb der Matte 5 oder ist an der Außenfläche der Matte 5 festgelegt, und einen externen Abschnitt 3 auf, d.h. es ist ein Leitungsabschnitt außerhalb der Matte 5.

In Fig. 2 ist eine erfindungsgemäße Ausführungsform dargestellt, die das Messgerät 1 beinhaltet. Das Messgerät 1 kann dabei in die Matte 5 integriert oder auf der Matte 5 aufgebracht sein. Anstelle der integrierten Bauweise kann das Messgerät 1 auch außerhalb der Matte 5 angeordnet sein. Die externen Leitungsabschnitte 3 können mit der Matte 5 fest verbunden sein oder abnehmbar festgelegt sein. Die Festlegung kann beispielsweise durch Druckknöpfe erfolgen. Dargestellt ist die Festlegung der externen Leitungsabschnitte 3 in den Eckenbereichen einer rechteckigen Matte 5.

Alternativ können die externen Leitungsabschnitte 3 auch an beliebiger Stelle festgelegt sein. Auch ist es möglich, die Matte 5 in einer anderen als der dargestellten rechteckigen Form auszubilden. Ist das Messgerät 1 integraler Bestandteil der Matte 5, kann es mit sich oder an einer beliebigen Stelle der Matte 5 angeordnet sein. Das Subjekt S ist durch die Messmittel 4, den jeweils externen Leitungsabschnitt 3 und den jeweils internen Leitungsabschnitt 2 mit dem Messgerät 1 verbunden.

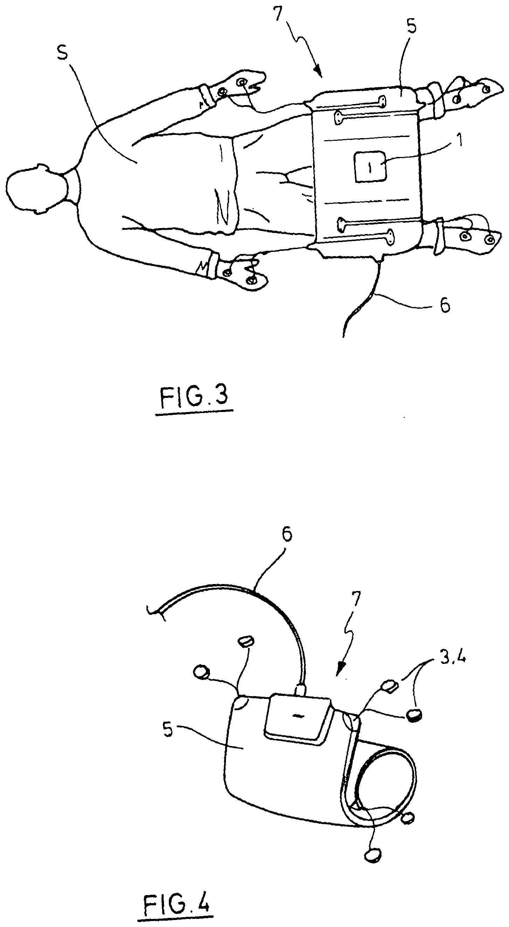

Fig. 3 zeigt die erste Ausführungsform der erfindungsgemäßen Vorrichtung zur Kontaktherstellung zwischen Messmittel 4 und Messgerät 1 bildlich. Im Gegensatz zur schematischen Darstellung der Figur 2 ist hier das Messgerät 1 nicht integraler Bestandteil der erfindungsgemäßen Matte 5, sondern das Messgerät 1 ist extern angeordnet. Das Messgerät (nicht dargestellt) wird durch eine Leitung 6 mit der Matte 5 verbunden. Die zusammengerollte oder zusammengelegte Matte 5 kann zum Beispiel an das Gehäuse des Messgerätes 1 angehängt werden und unterstützt somit den platzsparenden Transport, ohne dass sich die Leitungen 2, 3 verheddern, verknoten oder verdrehen können. Durch die Festlegung der externen Leitungsabschnitte 3 an den jeweiligen Eckenbereichen der Matte 5 wird der Anwender bzw. die Bedienungsperson intuitiv angeleitet, an welche Extremität der jeweils nächstliegende externe Leitungsabschnitt 4 mit seinen endseitigen Messmittel 4 zu verbinden ist.

Fig. 4 zeigt schematisch die erste Ausführungsform der erfindungsgemäßen Vorrichtung zur Kontaktherstellung 7 zwischen Messmittel 4 und Messgerät 1 in einem durch rollen zusammengelegten Zustand. Anstelle die Matte 5 zusammenzurollen um dadurch den zusammengelegten Zustand zu erreichen kann die Matte 5 auch durch Falten zusammengelegt sein. Die Matte 5 ist also derart ausgebildet, dass sie durch rollen wie in Fig 4. dargestellt oder durch falten (ohne Abbildung) zusammenlegbar ist.

In der Ausführungsvariante mit externen Leitungsabschnitten 3 und endseitig festgelegten Messmitteln 4 ist die erfindungsgemäße Matte 5 weiterhin derart ausgebildet, dass die externen Leitungsabschnitte 3 innerhalb der zusammengelegten Matte 5 aufgenommen und gegen herausfallen gesichert werden.

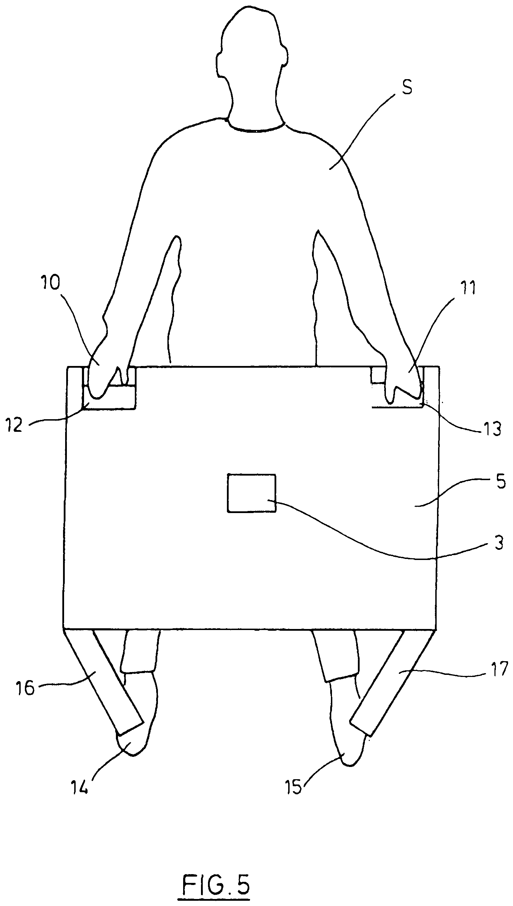

Fig. 5 zeigt das Subjekt S, das mit seinen Händen 10, 11 im Bereich der Matte 5 angeordnete Elektroden 12, 13 ergreift. Die Elektroden 12, 13 bilden hierbei jedenfalls teilweise die Messmittel 4 aus.

Zur Verbindung mit Füßen 14, 15 des Subjektes S sind an der Matte 5 Ausleger 16, 17 angeordnet. Im Bereich der Ausleger 16, 17 werden typischerweise Kabel angeordnet, die im Bereich der Ausleger 16, 17 positionierte Elektroden mit den im Bereich der Matte 5 angeordneten Messmitteln 3 verbinden. Die im Bereich der Ausleger 16, 17 positionierten Elektroden können als Klebeelektroden ausgebildet sein.

Vorzugsweise sind die Elektroden 12, 13 benachbart zu einem ersten Rand der Matte 5 angeordnet. Die Ausleger 16, 17 erstrecken sich vorzugsweise ausgehend von einem dem ersten Rand der Matte 5 gegenüberliegenden zweiten Rand.

Gemäß einer Ausführungsvariante wird die in Fig. 5 veranschaulichte Anordnung der Elektroden 12, 13 und der Ausleger 16, 17 vertauscht. Dies bedeutet, dass bei der Ausführungsvariante die Füße 14, 15 auf spezielle an der Matte 5 angeordnete Elektroden aufgesetzt werden und dass über die Ausleger 16, 17 die Hände 10, 11 elektrisch kontaktiert werden. Grundsätzlich wäre es auch denkbar, sowohl für die Hände 10, 11 als auch für die Füße 14, 15 vorgesehene Elektroden in die Matte 5 einzuarbeiten. Zur Anpassung an unterschiedliche Körpergrößen des Subjektes 1 erweist sich bei einer derartigen Ausführungsform aber eine veränderliche Größenausdehnung der Matte 5 als zweckmäßig.

Im Hinblick auf die konstruktive Gestaltung der Matte 5 besteht ein wesentliches konstruktives Merkmal darin, dass die Matte 5 Kabel zu verschiedenen Körperstellen des Subjektes S führt. Es werden somit insbesondere mehr als ein Kabel geführt. Zur Anpassung an die vorgesehene Anwendung weist die Matte 5 eine zweckmäßige Länge und Breite auf.

Gemäß einer bevorzugten Anwendung ist das Messgerät 1 als ein Gerät zur Erfassung und Auswertung einer mindestens bereichsweisen stofflichen Zusammensetzung des Körpers des Subjektes ausgebildet. Besonders bevorzugt ist bei einer Konkretisierung des Subjektes S als Mensch oder Patient an eine Anwendung bei einem BCA (Body Composition Analizer) gedacht. Mit Hilfe eines derartigen Gerätes erfolgt insbesondere eine Analyse, welche Gewichtsanteile der menschliche Körper z. B. an Fett, Muskelmasse sowie Knochenmasse besitzt. Die Auswertung kann sich aber auch auf eine Vielzahl anderer oder zusätzlicher Parameter beziehen.

Typischerweise weist die Matte 5 wenigstens zwei Ecken auf. Bevorzugt handelt es sich um wenigstens drei Ecken, typischerweise um vier Ecken.

Bei den bereits erläuterten Ausführungsbeispielen wurde teilweise definiert, dass das Messmittel 4 an der Matte 5 festlegbar ist. Eine derartige Festlegbarkeit umfasst jede zumindest für die Durchführung des Messvorganges vorliegende mechanische Verbindung zwischen dem Messmittel 4 und der Matte 5. Umfasst sind hiervon insbesondere dauerhafte Verklebungen oder Verpressungen oder eine lediglich zeitweise Verbindung über lösbare Verbindungselemente, beispielsweise Klettverschlüsse oder Steckkupplungen.

Der vorstehend mehrfach benutzte Begriff der Matte 5 umfasst zum einen weiche und/oder flexible und/oder elastische Materialien. Alternativ ist aber auch eine starre brettartige oder plattenartige Ausführungsform realisierbar. Darüber hinaus ist auch an eine Ausbildung aus mehreren miteinander verbundenen jeweils starren Segmenten gedacht, die zusammengefaltet werden können und über oder unter dem Subjekt positioniert werden. Eine derartige Ausführungsform kann ähnlich zu einer Panzerkette realisiert sein.

Alternativ zur Verwendung einer ein elektrisches Signal übertragenden Leitung 6 ist auch an die Verwendung von drahtlosen Verbindungen gedacht, beispielsweise die Verwendung einer Funkverbindung. Gemäß einer besonderen Ausführungsform ist die Matte 5 mit einer Anzeigeeinrichtung versehen. Die Anzeigeeinrichtung kann beispielsweise einen jeweiligen Betriebsstatus visualisieren. Darüber hinaus ist es möglich, über die Anzeige Funktions- oder Bedienfehler zu visualisieren. Auch eine Messgeräte-Bedienung und/oder eine Messergebnis-Anzeige (GUI) sind möglich. Hinsichtlich der Betriebssicherheit können Hinweise auf Funktions- oder Bedienfehler auch akustisch ausgegeben werden.

Eine Kennzeichnung oder Auswahl der Positionierung der Vorrichtung 7 kann zum Beispiel automatisch über eine Positionserkennung erfolgen. Alternativ ist eine entsprechende Kennzeichnung oder Auswahl auch manuell durch einen Tastendruck an der Matte 5 oder am Messgerät 1 möglich.

## Patentansprüche

1. Vorrichtung (7) zur messtechnischen Kontaktherstellung zwischen einem Patienten und einem Messgerät (1), aufweisend Messmittel (4) zur Anbindung an den Patienten und Leitungen (2, 3), an denen die Messmittel (4) festgelegt sind, wobei die Leitungen (2, 3) derart ausgebildet sind, dass die durch die Messmittel (4) erfassten Messgrößen an ein Messgerät (1) leitbar sind sowie bei der eine Matte (5) mit den Leitungen (2, 3) derart verbunden ist, dass wenigstens teilweise ein interner Leitungsabschnitt (2) gebildet ist, **dadurch gekennzeichnet, dass** die Messmittel als Elektroden (12, 13) zur Durchführung einer Bioimpedanzmessung ausgebildet sind und dass eine Elektrode (12, 13) für jede Hand (10, 11) des Patienten und eine Elektrode für jeden Fuß (14, 15) des Patienten in die Matte (5) eingearbeitet sind.

2. Vorrichtung (7) nach Anspruch 1, **dadurch gekennzeichnet, dass** wenigstens ein interner Leitungsabschnitt (2) an einer Außenfläche der Matte (5) festlegbar ist.

3. Vorrichtung (7) nach Anspruch 1, **dadurch gekennzeichnet, dass** wenigstens ein interner Leitungsabschnitt (2) innerhalb der Matte (5) angeordnet ist.

4. Vorrichtung (7) nach Anspruch 1, **dadurch gekennzeichnet, dass** wenigstens ein interner Leitungsabschnitt (2) integraler Bestandteil der Matte (5) ist.

5. Vorrichtung (7) nach einem der vorgehenden Ansprüche, **dadurch gekennzeichnet, dass** wenigstens ein externer Leitungsabschnitt (3) von dem internen Leitungsabschnitt (2) lösbar ist.

6. Vorrichtung (7) nach einem der vorgehenden Ansprüche, **dadurch gekennzeichnet, dass** die Matte (5) wenigstens zwei Ecken aufweist, in deren Bereichen die externen Leitungsabschnitte (3) angeordnet sind.

7. Vorrichtung (7) nach einem der vorgehenden Ansprüche, **dadurch gekennzeichnet, dass** wenigstens ein Messmittel (4) an der Matte (5) festlegbar ist.

8. Vorrichtung (7) nach Anspruch 7, **dadurch gekennzeichnet, dass** wenigstens ein Messmittel (4) integraler oder lösbarer Bestandteil eines Abschnittes der Matte (5) ist.

9. Vorrichtung (7) nach einem der Ansprüche 6 bis 8, **dadurch gekennzeichnet, dass** das wenigstens eine Messmittel (4) in einem Eckenbereichsabschnitt der Matte (5) angeordnet ist

10. Vorrichtung (7) nach Anspruch 1, **dadurch gekennzeichnet, dass** die Matte (5) durch Rollen oder Falten zusammenlegbar ist.

11. Vorrichtung (7) nach Anspruch 10, **dadurch gekennzeichnet, dass** die Matte (5) derart zusammenlegbar ist, dass die externen Leitungsabschnitte (3, 4) in der zusammengelegten Matte angeordnet und gegen Herausfallen gesichert sind.

12. Vorrichtung (7) nach einem der vorgehenden Ansprüche, **dadurch gekennzeichnet, dass** die Matte (5) eine integrierte Energieversorgung, vorzugsweise eine Stromversorgung durch eine Batterie oder einen Akkumulator aufweist.

13. Vorrichtung (7) nach einem der vorgehenden Ansprüche, **dadurch gekennzeichnet, dass** ein Messgerät (1) integraler Bestandteil der Matte (5) ist.

14. Vorrichtung (7) nach einem der Ansprüche 1 bis 12, **dadurch gekennzeichnet, dass** ein Messgerät (1) an der Oberfläche der Matte (5) festlegbar ist.

15. Vorrichtung (7) nach einem der Ansprüche 1 bis 12 **gekennzeichnet durch** eine Matte (5) aufweisend Anschlussmittel (6) geeignet zum Anschluss eines von der Matte (5) beabstandeten Messgerätes (1).

16. Vorrichtung (7) nach einem der vorgehenden Ansprüche, **dadurch gekennzeichnet, dass** die Elektroden durch Klebeelektroden oder Handgriffelektroden oder Fußkontaktelektroden oder federgespannte Klemmelektroden gebildet sind.

17. Verwendung der Vorrichtung (7) nach einem der vorgehenden Ansprüche durch Auflegen auf oder Unterlegen unter den zu messenden Patienten.

18. Messsystem zur Messung von Bioimpedanz aufweisend eine Vorrichtung (7) nach einem der Ansprüche 1 bis 16.

19. Vorrichtung nach einem der Ansprüche 1 bis 16, **dadurch gekennzeichnet**, das eine Bedienung und/oder eine Anzeige eines externen oder in die Matte integrierten Messgerätes an/durch die Matte erfolgt.

## Claims

1. Device (7) for establishing contact between a patient and a measuring unit (1) by way of measurement technology, having measuring means (4) for connection to the patient and leads (2, 3) to which the measuring means (4) are fastened, wherein the leads (2, 3) are designed in such a way that the measurement variables recorded by the measuring means (4) can be conducted to a measuring unit (1), and in which a mat (5) is connected to the leads (2, 3) in such a way that at least partially an internal lead section (2) is formed, **characterized in that** the measuring means are designed as electrodes (12, 13) for carrying out a bioimpedance measurement, and **in that** one electrode (12, 13) for each hand (10, 11) of the patient and one electrode for each foot (14, 15) of the patient are incorporated in the mat (5).

2. Device (7) according to claim 1, **characterized in that** at least one internal lead section (2) can be fastened to an outer surface of the mat (5).

3. Device (7) according to claim 1, **characterized in that** at least one internal lead section (2) is arranged within the mat (5).

4. Device (7) according to claim 1, **characterized in that** at least one internal lead section (2) is an integral component of the mat (5).

5. Device (7) according to any of the preceding claims, **characterized in that** at least one external lead section (3) is detachable from the internal lead section (2).

6. Device (7) according to any of the preceding claims, **characterized in that** the mat (5) has at least two corners, in the regions of which the external lead sections (3) are arranged.

7. Device (7) according to any of the preceding claims, **characterized in that** at least one measuring means (4) can be fastened to the mat (5).

8. Device (7) according to claim 7, **characterized in that** at least one measuring means (4) is an integral or detachable component of a section of the mat (5).

9. Device (7) according to any of claims 6 to 8, **characterized in that** the at least one measuring means (4) is arranged in a corner region section of the mat (5).

10. Device (7) according to claim 1, **characterized in that** the mat (5) is collapsible by rolling or folding.

11. Device (7) according to claim 10, **characterized in that** the mat (5) is collapsible in such a way that the external lead sections (3, 4) are arranged in the collapsed mat and are secured against falling out.

12. Device (7) according to any of the preceding claims, **characterized in that** the mat (5) has an integrated power supply, preferably a current supply by way of a battery or an accumulator.

13. Device (7) according to any of the preceding claims, **characterized in that** a measuring unit (1) is an integral component of the mat (5).

14. Device (7) according to any of claims 1 to 12, **characterized in that** a measuring unit (1) can be fastened to the surface of the mat (5).

15. Device (7) according to any of claims 1 to 12, **characterized by** a mat (5) having connection means (6) suitable for connecting a measuring unit (1) that is arranged at a distance from the mat (5).

16. Device (7) according to any of the preceding claims, **characterized in that** the electrodes are formed by adhesive electrodes or hand-grip electrodes or foot-contact electrodes or spring-loaded clamp electrodes.

17. Use of the device (7) according to any of the preceding claims by placing it onto or below the patient to be measured.

18. Measuring system for measuring bioimpedance, having a device (7) according to any of claims 1 to 16.

19. Device according to any of claims 1 to 16, **characterized in that** a control and/or a display of an external measuring unit or of a measuring unit integrated in the mat takes place on/through the mat.

## Revendications

1. Dispositif (7) pour établir, selon la technique de mesure, le contact entre un patient et un appareil de mesure (1), présentant des moyens de mesure (4), destinés à être reliés au patient et des conducteurs (2, 3) auxquels sont fixés les moyens de mesure (4), sachant que les conducteurs (2, 3) sont conçus de manière à ce que les valeurs de mesure saisies par les moyens de mesure (4) puissent être transmises à un appareil de mesure (1), et qu'un matelas (5) est relié aux conducteurs (2, 3) de manière à ce qu'une section de conduction interne (2) soit formée au moins partiellement,
**caractérisé en ce que** les moyens de mesure sont réalisés sous la forme d'électrodes (12, 13) pour l'exécution d'une mesure d'impédance biologique, et qu'une électrode (12, 13) pour chaque main (10, 11) du patient et une électrode pour chaque pied (14, 15) du patient sont incorporées dans le matelas (5).

2. Dispositif (7) selon la revendication 1, **caractérisé en ce qu'**au moins une section de conduction interne (2) peut être fixée sur une face extérieure du matelas (5).

3. Dispositif (7) selon la revendication 1, **caractérisé en ce qu'**au moins une section de conduction interne (2) est disposée à l'intérieur du matelas (5).

4. Dispositif (7) selon la revendication 1, **caractérisé en ce qu'**au moins une section de conduction interne (2) est partie intégrante du matelas (5).

5. Dispositif (7) selon l'une des revendications précédentes,
**caractérisé en ce qu'**au moins une section de conduction externe (3) peut être séparée de la section de conducteur interne (2).

6. Dispositif (7) selon l'une des revendications précédentes,
**caractérisé en ce que** le matelas (5) est doté d'au moins deux angles, dans la région desquels les sections de conduction externes (3) sont disposées.

7. Dispositif (7) selon l'une des revendications précédentes,
**caractérisé en ce qu'**au moins un moyen de mesure (4) peut être relié fixement au matelas (5).

8. Dispositif (7) selon la revendication 7, **caractérisé en ce qu'**au moins un moyen de mesure (4) est partie intégrante ou amovible d'une zone du matelas (5).

9. Dispositif (7) selon l'une des revendications 6 à 8,
**caractérisé en ce qu'**au moins un moyen de mesure (4) est disposé dans une section d'angle du matelas (5).

10. Dispositif (7) selon la revendication 1, **caractérisé en ce que** le matelas (5) peut être mis en paquet roulé ou plié.

11. Dispositif (7) selon la revendication 10, **caractérisé en ce que** le matelas (5) peut être mis en paquet de manière à ce que les sections de conduction externes (3, 4) soient disposées dans le matelas (5) en paquet et ne risquent pas de tomber au dehors.

12. Dispositif (7) selon l'une des revendications précédentes,
**caractérisé en ce que** le matelas (5) est doté d'une alimentation en énergie intégrée, de préférence d'une alimentation en courant au moyen d'une batterie ou d'un accumulateur.

13. Dispositif (7) selon l'une des revendications précédentes,
**caractérisé en ce que** l'appareil de mesure (1) est partie intégrante du matelas (5).

14. Dispositif (7) selon l'une des revendications 1 à 12,
**caractérisé en ce qu'**un appareil de mesure (1) peut être fixé sur la surface du matelas (5).

15. Dispositif (7) selon l'une des revendications 1 à 12,
**caractérisé par** un matelas (5) doté d'un moyen de raccordement (6) apte au raccordement d'un appareil de mesure (1) distancé du matelas (5).

16. Dispositif (7) selon l'une des revendications précédentes,
**caractérisé en ce que** les électrodes sont formées par des électrodes adhésives ou des électrodes à poignée ou par des électrodes contact pieds ou des électrodes à crampon commandées par ressort.

17. Utilisation du dispositif (7) selon l'une des revendications précédentes par placement sur ou sous le patient devant être soumis aux mesures.

18. Système de mesure d'impédance biologique présentant un dispositif selon l'une des revendications 1 à 16.

19. Dispositif selon l'une des revendications 1 à 16, **caractérisé en ce qu'**une commande et / ou un affichage d'un appareil de mesure extérieur ou intégré dans le matelas s'effectuent sur / à travers le matelas.
